# EUROPEAN PATENT APPLICATION

(11) **EP 4 497 817 A1**
(43) Date of publication of application: **29.01.2025**
(21) Application number: 23774235.8
(22) Date of filing: 27.01.2023
(51) Int. Cl.: C12N 1/00, C12M 1/00

(54) **CELL ISOLATION METHOD AND CELL ISOLATION APPARATUS**

(30) Priority: 23.03.2022 JP 2022047028
(71) Applicant: FUJIFILM Corporation, Tokyo 106-8620 (JP)
(72) Inventor: TAKAHASHI, Hiroaki, Ashigarakami-gun, Kanagawa 258-8577 (JP); WAKABAYASHI, Akira, Ashigarakami-gun, Kanagawa 258-8577 (JP); SHIGESADA, Keiji, Ashigarakami-gun, Kanagawa 258-8577 (JP)
(74) Representative: Hoffmann Eitle
(86) International application number: PCT/JP2023/002758
(87) International publication number: WO 2023/181636

(57) **Abstract**

A cell isolation method of isolating a cell from a biological tissue includes cutting processing of pressing a blade against a blade receiver and pressing and cutting the biological tissue disposed on the blade receiver.

## Description

### BACKGROUND

### Technical Field

The disclosed technology relates to a cell isolation method and a cell isolation device.

### Related Art

Regarding a technique for isolating cells from a biological tissue, following techniques are known. For example, JP2021-525550A discloses a system including a pretreatment chamber that includes a tissue disruption device that is driven by an actuator and mechanically disrupts a tissue, and a processing chamber configured to collect a suspension of a biological material from the pretreatment chamber.

JP2014-534817A discloses a tissue morcellation tool including a compartment for a tissue sample, a cut surface at one end of the compartment, and a sterilized sealed container. The cut surface separates the compartment from the container such that the tissue sample passing through the cut surface is deposited in the container.

Regenerative medicine is a medical care for repairing and regenerating organs or tissues that have been lost due to diseases, injuries, and the like, using artificially cultured cells. For example, a meniscal preservation treatment of administering a cell floating liquid formed of an autologous mesenchymal stem cell derived from a synovial membrane (synovial stem cell) to a patient with meniscal injury is known. The synovial stem cells are obtained by performing a cell isolation processing on the synovial membrane collected from the patient.

In the cell isolation processing in the related art, since the process of a manual work is mainly done by skilled workers, it is not easy to improve manufacturing capacity, and it is estimated that the manufacturing capacity is insufficient for the demand for cell products. Therefore, it is desired to automate the cell isolation processing in order to improve the manufacturing capacity of the cell products. In the automation of the cell isolation processing, it is important to suppress damage to cells and ensure a high survival rate of cells after the treatment.

### SUMMARY

The disclosed technique has been made in view of the above points, and an object thereof is to ensure a high survival rate in cells obtained by performing the cell isolation processing.

A cell isolation method according to an aspect of the disclosed technology is a cell isolation method of isolating a cell from a biological tissue, and the cell isolation method includes performing cutting processing of pressing a blade against a blade receiver and pressing and cutting the biological tissue disposed on the blade receiver, by a mechanical operation.

The cutting processing may be performed inside a sealed container. In the cutting processing, the biological tissue may be cut while a cutting position of the biological tissue by the blade is changed. The cell isolation method according to the disclosed technology may include pressing and cutting the biological tissue by a translational movement of the blade. A pressing force in a case of pressing the blade against the blade receiver may be controlled to a minimum magnitude required for cutting the biological tissue.

The cell isolation method according to the disclosed technology may include a first step of dividing the biological tissue accommodated in a container into small pieces by the cutting processing; and a second step of introducing a first processing liquid into the container to decompose the small pieces of the biological tissue in the container. In the first step, the biological tissue may be divided while a cutting position of the biological tissue by the blade is changed. In the first step, the biological tissue may be divided in a state where the blade is immersed in a liquid introduced into the container. In the second step, the blade may be rotated in a state where the blade is immersed in the first processing liquid. In the second step, the first processing liquid may be controlled to an optimum temperature. In the first step and the second step, an inside of the container is preferably maintained in a sealed state. The cell isolation method according to the disclosed technology may further include a third step of introducing a second processing liquid into the container to stop decomposition of the biological tissue after the second step.

A cell isolation device according to another aspect of the disclosed technology is a cell isolation device for isolating a cell from a biological tissue, and the cell isolation device includes a blade for cutting the biological tissue; a blade receiver against which the blade is pressed; and a driving unit that drives the blade such that the blade is pressed against the blade receiver.

The driving unit may drive the blade such that the biological tissue is pressed and cut by a translational movement of the blade. The driving unit may drive the blade such that a cutting position of the biological tissue by the blade is changed. The driving unit may change the cutting position by a rotation operation in which a direction of the translational movement of the blade is a rotation axis direction. The driving unit may repeatedly perform a series of operations including an operation of pressing the blade against the blade receiver, an operation of separating the blade from the blade receiver, and an operation of changing a relative position between the blade and the blade receiver. The driving unit may control a pressing force in a case of pressing the blade against the blade receiver to a minimum magnitude required for cutting the biological tissue.

The blade receiver may include a first member having elasticity, and a second member that is stacked on the first member and has stiffness higher than the first member, and the blade may be pressed against the second member. The second member may be thinner than the first member. The cell isolation device according to the disclosed technology may include an adhesion suppressing member for suppressing adhesion of the biological tissue to the blade. The adhesion suppressing member may have an inclined part that is provided on a side surface of the blade and is inclined toward a blade tip of the blade.

The cell isolation device according to the disclosed technology may include a container in which the biological tissue is accommodated; and a liquid feeding portion that transfers a first processing liquid for decomposing the biological tissue cut by the blade in the container, to the container. The container may include a first port for introducing a liquid or a gas containing the first processing liquid into the container, a second port for discharging a processed liquid from the container, and a third port for an air vent. The cell isolation device according to the disclosed technology may further include a switching mechanism that switches the liquid or the gas to be introduced into the container via the first port. The cell isolation device according to the disclosed technology may further include a mechanism that inclines the container such that discharge of a processing liquid from the second port is promoted. The cell isolation device according to the disclosed technology may further include a tubular cap that covers the container; and a shaft portion that is inserted into the cap, and of which one end is attached to the blade and the other end is attached to the driving unit. The blade may be attached to the shaft portion via a grip portion that grips the blade by point contact or line contact with respect to the blade.

The cell isolation device according to the disclosed technology may further include a sealing member that seals a gap formed between the cap and the shaft portion. The sealing member is preferably composed of a member having flexibility. The sealing member may have a large-diameter portion and a small-diameter portion that are arranged along an extension direction of the shaft portion, and the small-diameter portion may be buried under the large-diameter portion in accordance with a translational movement along the extension direction of the shaft portion. The sealing member may have a crease for regulating a folding position in a case where the small-diameter portion is buried under the large-diameter portion.

The cell isolation device according to the disclosed technology may further include a temperature control mechanism that controls the first processing liquid accommodated in the container to an optimum temperature.

The cell isolation device according to the disclosed technology may further include a control unit that controls the driving unit and the liquid feeding portion such that a first step of dividing the biological tissue accommodated in the container into small pieces by cutting the biological tissue using the blade, and a second step of introducing the first processing liquid into the container to decompose the divided biological tissue in the container are sequentially executed. The control unit may control the driving unit such that the blade is rotated in a state where the blade is immersed in the first processing liquid, in the second step.

The control unit may control the liquid feeding portion such that a third step of introducing a second processing liquid into the container to stop decomposition of the biological tissue is executed after the second step. The control unit may control the liquid feeding portion such that a fourth step of discharging a processing liquid containing the cell isolated from the biological tissue, from the container is executed after the third step.

According to the disclosed technology, it is possible to ensure a high survival rate in cells obtained by performing the cell isolation processing.

### BRIEF DESCRIPTION OF THE DRAWINGS

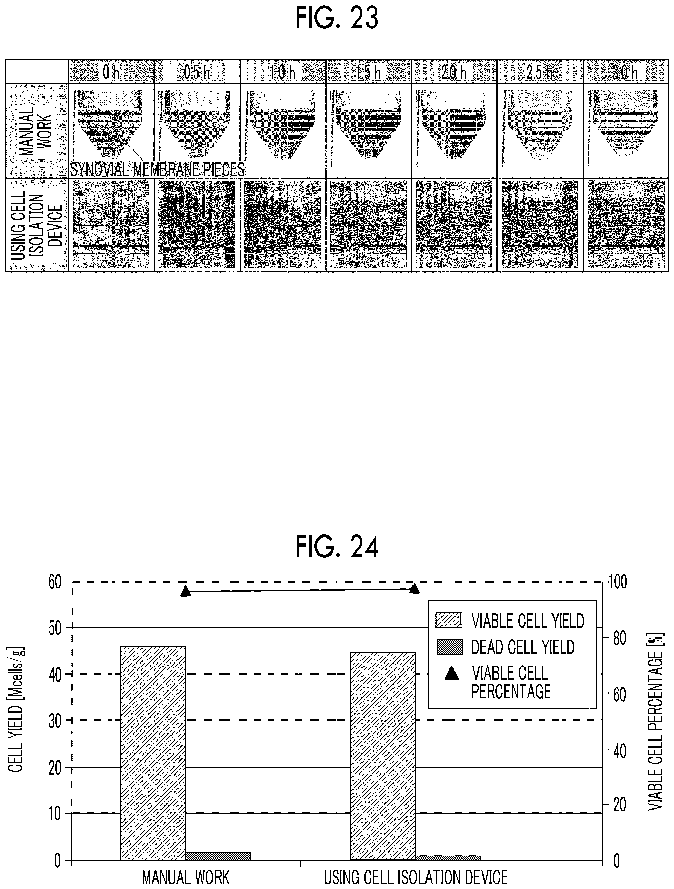

Fig. 1 is a diagram schematically illustrating an example of a basic configuration of a cell isolation device according to an embodiment of the disclosed technology.
Fig. 2 is a plan view illustrating an example of a configuration of a processing module according to the embodiment of the disclosed technology.
Fig. 3A is a plan view illustrating an example of a configuration of a processing container according to the embodiment of the disclosed technology.
Fig. 3B is a sectional view taken along line 3B-3B in Fig. 3A.
Fig. 4A is a plan view illustrating an example of a configuration of a cap according to the embodiment of the disclosed technology.
Fig. 4B is a sectional view taken along line 4B-4B in Fig. 4A.
Fig. 5 is a perspective view illustrating an example of a configuration of a shaft portion according to the embodiment of the disclosed technology.
Fig. 6A is a diagram illustrating a state where a blade according to the embodiment of the disclosed technology is gripped by a grip portion.
Fig. 6B is a perspective view illustrating an example of a configuration of a second portion of the grip portion according to the embodiment of the disclosed technology.
Fig. 7A is a perspective view illustrating an example of a configuration of a sealing member 18.
Fig. 7B is a sectional view illustrating an example of a configuration of the sealing member 18.
Fig. 8A is a sectional view illustrating an engaging part between a cap and a shaft portion in a case where the sealing member is not provided.
Fig. 8B is a sectional view illustrating the engaging part between the cap and the shaft portion in a case where the sealing member is not provided.
Fig. 9A is a sectional view illustrating the engaging part between the cap and the shaft portion in a case where the sealing member is provided.
Fig. 9B is a sectional view illustrating the engaging part between the cap and the shaft portion in a case where the sealing member is provided.
Fig. 10 is a diagram illustrating an example of a configuration of a driving unit according to the embodiment of the disclosed technology.
Fig. 11 is a perspective view illustrating an example of a configuration of a blade receiver according to the embodiment of the disclosed technology.
Fig. 12 is a diagram illustrating an example of an operation sequence of a processing module in a first step according to the embodiment of the disclosed technology.
Fig. 13 is a diagram illustrating an example of an aspect of a change in a relative position between the blade and the blade receiver according to the embodiment of the disclosed technology.
Fig. 14 is a diagram illustrating an example of an operation of a processing module in a second step according to the embodiment of the disclosed technology.
Fig. 15 is a diagram illustrating an example of a hardware configuration of a control unit according to the embodiment of the disclosed technology.
Fig. 16 is a flowchart illustrating an example of a flow of a treatment executed by a processor according to the embodiment of the disclosed technology executing a processing program.
Fig. 17 is a diagram illustrating an example of a configuration of an adhesion suppressing member according to the embodiment of the disclosed technology.
Fig. 18 is a perspective view illustrating an example of a configuration of a temperature control mechanism according to the embodiment of the disclosed technology.
Fig. 19 is a diagram illustrating an example of a configuration of an inclination mechanism according to the embodiment of the disclosed technology.
Fig. 20 is a diagram illustrating an example of an aspect of inclination of the driving unit and the processing module according to the embodiment of the disclosed technology.
Fig. 21A is an image of a synovial membrane divided into small pieces by using the cell isolation device according to the embodiment of the disclosed technology.
Fig. 21B is an image of the synovial membrane divided into small pieces by a manual work.
Fig. 22A is a histogram illustrating a size distribution of the synovial membrane divided into small pieces by using the cell isolation device according to the embodiment of the disclosed technology.
Fig. 22B is a histogram illustrating a size distribution of the synovial membrane divided into small pieces by a manual work in the related art.
The upper row of Fig. 23 is an image illustrating a state of decomposition of the synovial membrane by the manual work in the related art, and the lower row of Fig. 23 is an image illustrating a state of decomposition of the synovial membrane by the cell isolation device according to the embodiment of the disclosed technology.
Fig. 24 is a graph illustrating a yield and a viable cell percentage of cells isolated from the synovial membrane in a case of being performed by a manual work and in a case of being performed by using the cell isolation device according to the embodiment of the disclosed technology.

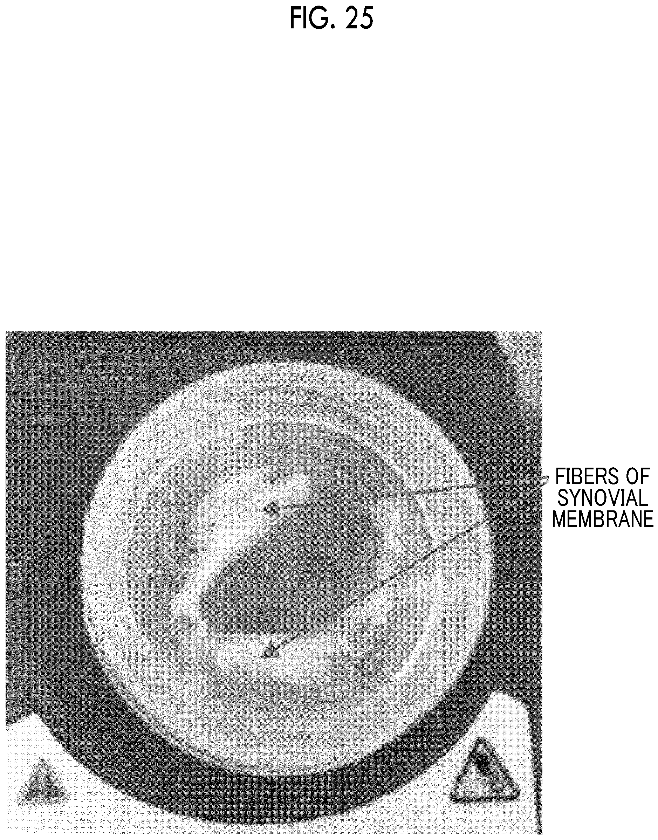

Fig. 25 is an image illustrating a state of the synovial membrane in a case where division processing of the synovial membrane is performed using a rotary blade.

### DETAILED DESCRIPTION

Hereinafter, an example of an embodiment of the disclosed technology will be described with reference to the drawings. It should be noted that the same or equivalent constituent elements and portions in the drawings are assigned by the same reference numerals, and overlapping descriptions will be omitted.

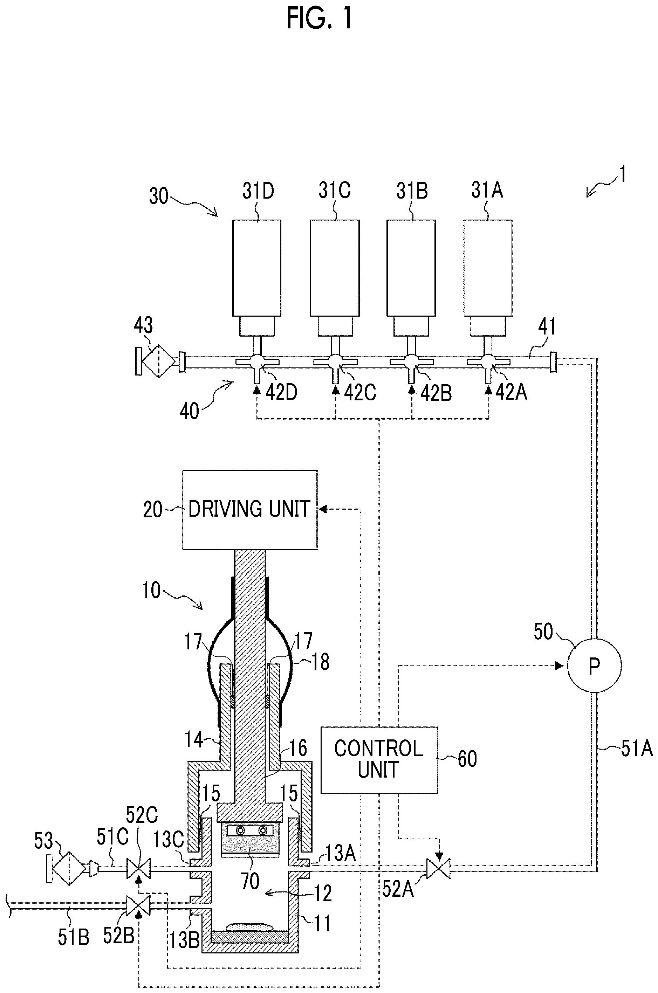

Fig. 1 is a diagram schematically illustrating an example of a basic configuration of a cell isolation device 1 according to an embodiment of the disclosed technology. The cell isolation device 1 is a device for isolating cells from a biological tissue, and includes a processing module 10, a driving unit 20, a processing liquid storage portion 30, a switching mechanism 40, a liquid feeding portion 50, and a control unit 60. The processing module 10 includes a processing container 11 that accommodates the biological tissue as a processing target. The processing container 11 is an example of a "container" in the disclosed technique.

The cell isolation device 1 has a function of dividing the biological tissue into small pieces in the processing container 11, and a function of introducing a processing liquid for decomposing the divided biological tissue in the processing container 11, into the processing container 11. That is, the cell isolation device 1 sequentially executes a first step of dividing the biological tissue accommodated in the processing container 11 into small pieces and a second step of introducing a first processing liquid into the processing container 11 to decompose the divided biological tissue in the processing container 11. By decomposing the biological tissue, cells contained in the biological tissue are isolated.

The cell isolation device 1 executes a third step of introducing a second processing liquid into the processing container 11 after the second step to stop the decomposition of the biological tissue. As the first processing liquid, for example, an enzyme solution can be used. As the second processing liquid, for example, a culture medium can be used. In addition, the cell isolation device 1 executes a fourth step of discharging the processing liquid containing the cells isolated from the biological tissue, from the processing container 11 after the third step. Hereinafter, the details of each of the above-described components of the cell isolation device 1 will be described.

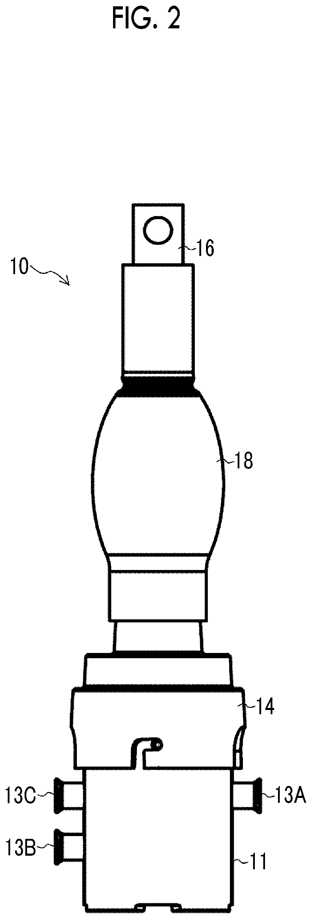

Fig. 2 is a plan view illustrating an example of a configuration of the processing module 10. The processing module 10 includes the processing container 11, a cap 14, a shaft portion 16, and a sealing member 18.

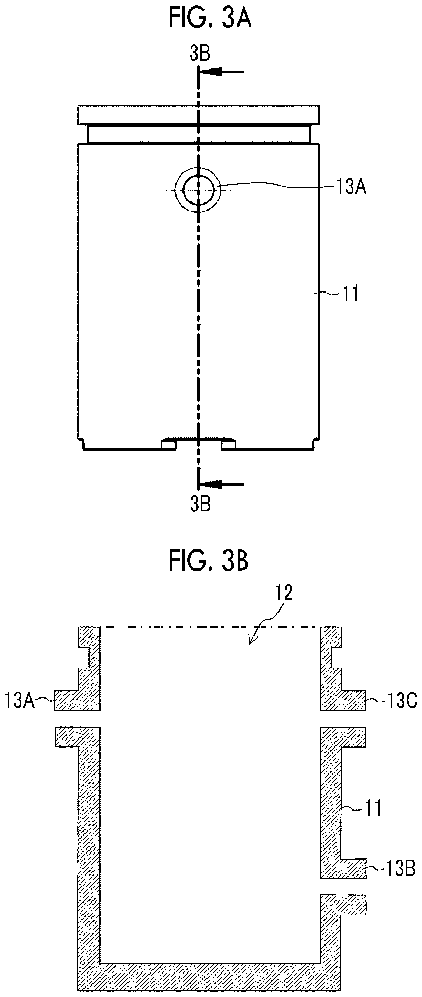

Fig. 3A is a plan view illustrating an example of a configuration of the processing container 11. Fig. 3B is a sectional view taken along line 3B-3B in Fig. 3A. The processing container 11 has a processing space 12 in which the biological tissue is accommodated. Through the first step and the second step, the state in which the biological tissue is accommodated in the processing container 11 is maintained. In addition, through the first step and the second step, the processing space 12 is maintained in a sealed state. A material of the processing container 11 is not particularly limited, but it is preferable that the processing container 11 is made of a light-transmitting member such that a situation of the inside of the processing space 12 can be visible from the outside. As the material of the processing container 11, for example, a resin such as polycarbonate or polystyrene can be used.

A first port 13A, a second port 13B, and a third port 13C are provided on side surfaces of the processing container 11. The first port 13A is a port for introducing various processing liquids or gases (air) stored in the processing liquid storage portion 30 into the processing container 11. The second port 13B is a port for discharging the processed liquid from the processing container 11. The third port 13C is a port for an air vent. The first port 13A, the second port 13B, and the third port 13C are formed by integral molding with the processing container 11, respectively, and are integrated with the processing container 11. That is, there is no seam between the first port 13A, second port 13B, and third port 13 and the processing container 11. Accordingly, it is possible to prevent the liquid accommodated in the processing container 11 from leaking. In addition, each of the first port 13A, the second port 13B, and the third port 13C does not have a portion that protrudes to the inside of the processing container 11. That is, an interior wall of the processing container 11 is a smooth surface without unevenness. Accordingly, it is possible to avoid a dead space being formed in the processing space 12. In addition, the manufacturing of the processing container 11 is facilitated.

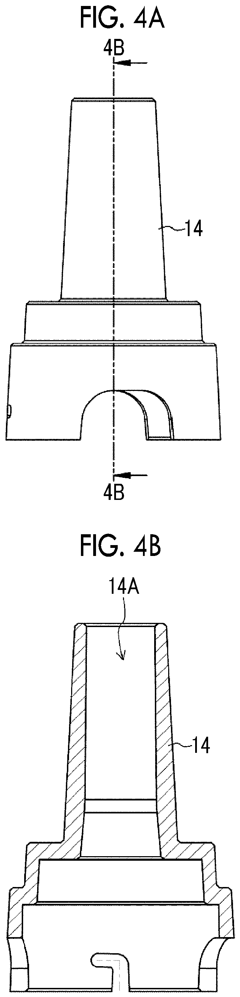

Fig. 4A is a plan view illustrating an example of the configuration of the cap 14. Fig. 4B is a sectional view taken along line 4B-4B in Fig. 4A. The cap 14 has a tubular structure having a through-hole 14A that penetrates from one end to the other end of the cap 14. The cap 14 covers the opening portion of the processing container 11. By covering the processing container 11 with the cap 14, it is possible to suppress a risk of a contamination source such as bacteria and viruses entering the processing space 12. In order to improve the sealing property of the processing space 12, it is preferable that an O-ring 15 (refer to Fig. 1) is provided in the engaging part between the cap 14 and the processing container 11.

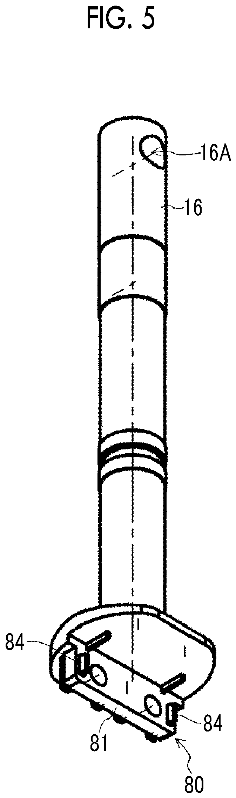

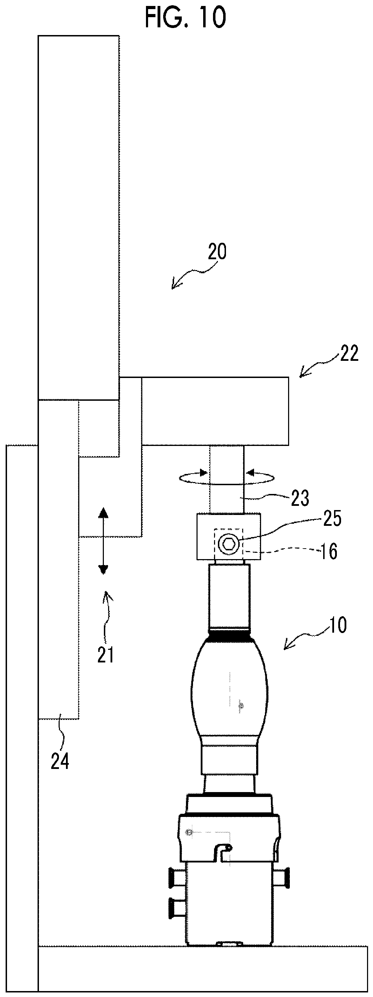

Fig. 5 is a perspective view illustrating an example of the configuration of the shaft portion 16. The shaft portion 16 is a rod-like member inserted into the through-hole 14A of the cap 14. In a state where the shaft portion 16 is inserted into the through-hole 14A of the cap 14, the shaft portion 16 can be moved in a translational movement along an extension direction (that is, in the up-down direction) and can be moved in a rotational movement about the extension direction as a rotation axis direction. A blade 70 (refer to Fig. 1) for cutting the biological tissue in the first step is attached to one end of the shaft portion 16. The other end of the shaft portion 16 is attached to the driving unit 20. A cylindrical hole 16A is provided in the other end of the shaft portion 16, and the shaft portion 16 is attached to the driving unit 20 using a bolt 25 (refer to Fig. 10) inserted into the cylindrical hole 16A.

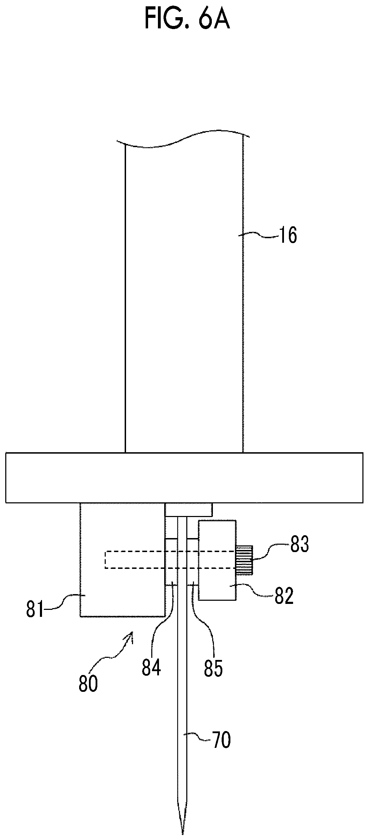

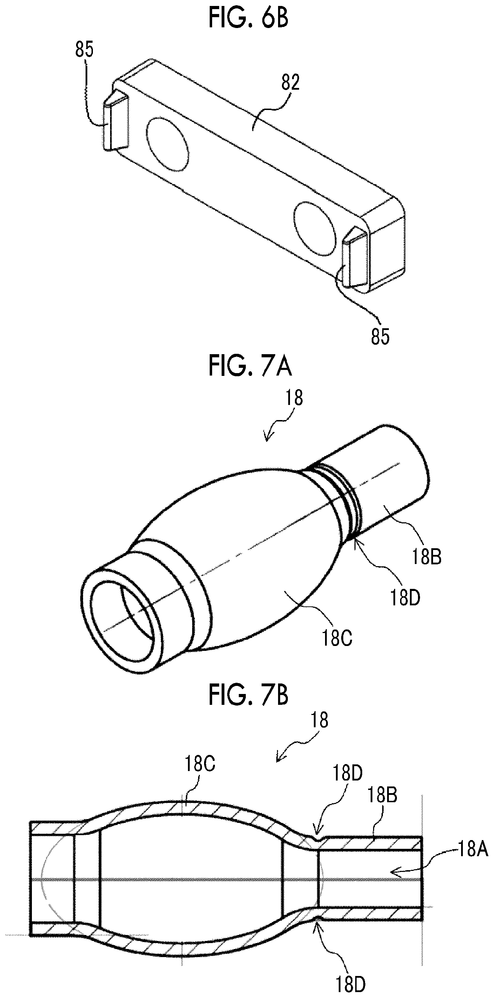

The blade 70 is attached to the shaft portion 16 via a grip portion 80. The blade 70 has a plate-like structure such as a razor blade, and a blade tip that comes into contact with the biological tissue is present in a straight line. Fig. 6A is a diagram illustrating a state where the blade 70 is gripped by the grip portion 80. The grip portion 80 includes a first portion 81 that is integrally provided with the body of the shaft portion 16, on a rear surface side of the blade 70, and a second portion 82 that is separately provided from the body of the shaft portion 16, on a front surface side of the blade 70. Fig. 6B is a perspective view illustrating an example of the configuration of the second portion 82 of the grip portion 80. The second portion 82 is fastened using a bolt 83 in a state where the blade 70 is sandwiched between the first portion 81 and the second portion 82. The first portion 81 and the second portion 82 have linear or point-like protrusions 84 and 85, respectively, and the protrusions 84 and 85 are in contact with the blade 70. That is, the contact between the grip portion 80 and the blade 70 is point contact or line contact. Accordingly, it is possible to reduce the contact area between the grip portion 80 and the blade 70. In a case where the contact area between the grip portion 80 and the blade 70 is large, the processing liquid is retained in the contact portion, and the blade 70 is likely to be corroded. By setting the contact between the grip portion 80 and the blade 70 to be point contact or line contact, it is possible to suppress the retention of the processing liquid in the contact portion and to prevent the corrosion of the blade 70. In order to improve the sealing property of the processing space 12, it is preferable that an O-ring 17 (refer to Fig. 1) is provided in the engaging part between the cap 14 and the shaft portion 16.

Figs. 7A and 7B are a perspective view and a sectional view illustrating an example of the configuration of the sealing member 18, respectively. The sealing member 18 has a tubular structure having a through-hole 18A that penetrates from one end to the other end of the sealing member 18. The sealing member 18 covers a region including a part of the shaft portion 16 and a distal end of the cap 14, at the engaging part between the shaft portion 16 and the cap 14, and seals a gap formed between the cap 14 and the shaft portion 16.

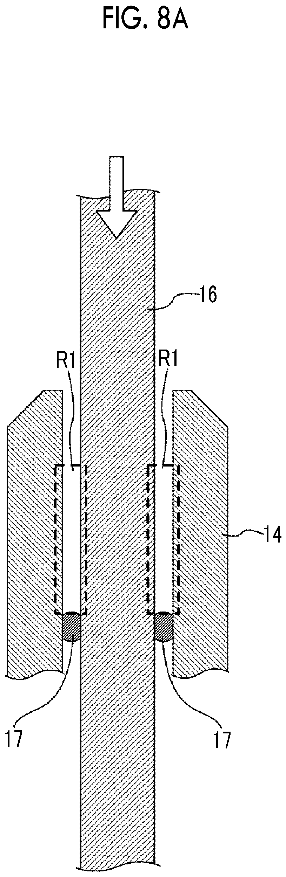

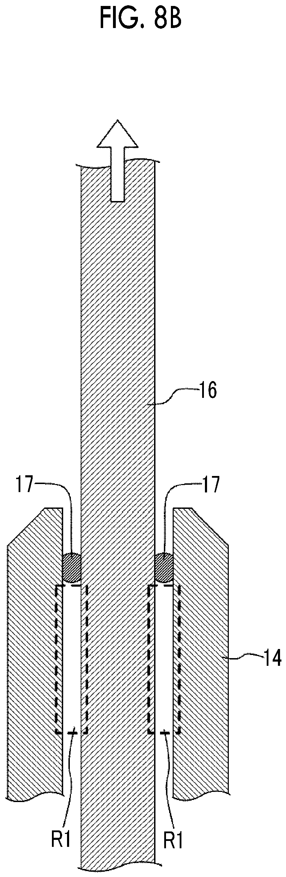

Here, Figs. 8A and 8B are sectional views illustrating the engaging part between the cap 14 and the shaft portion 16 in a state where the sealing member 18 is not provided. Fig. 8A illustrates a case where the shaft portion 16 that performs the translational movement is at a descending position, and Fig. 8B illustrates a case where the shaft portion 16 that performs the translational movement is at an ascending position. In the state illustrated in Fig. 8A, a region R1 surrounded by a dotted line, which is a gap between the cap 14 and the shaft portion 16, is separated from the processing space 12 of the processing container 11 by the O-ring 17 provided to the shaft portion 16. That is, the region R1 is exposed to the outside air, and has a risk of being contaminated by a contamination source such as bacteria and viruses. In a state illustrated in Fig. 8B, the contaminated region R1 communicates with the processing space 12, and there is a risk that the biological tissue in the processing space 12 is infected with the contamination source.

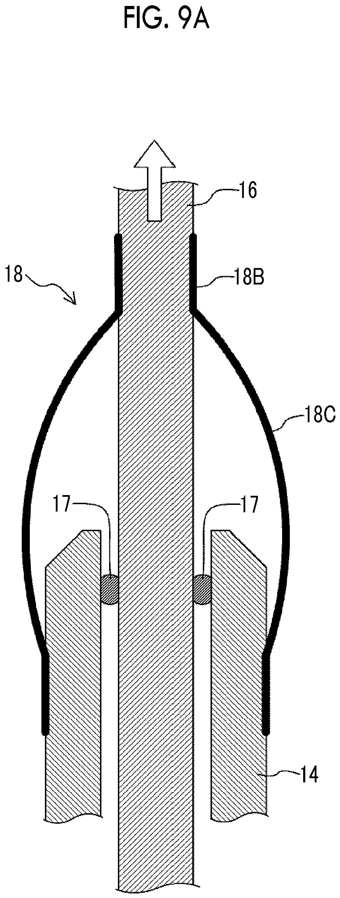

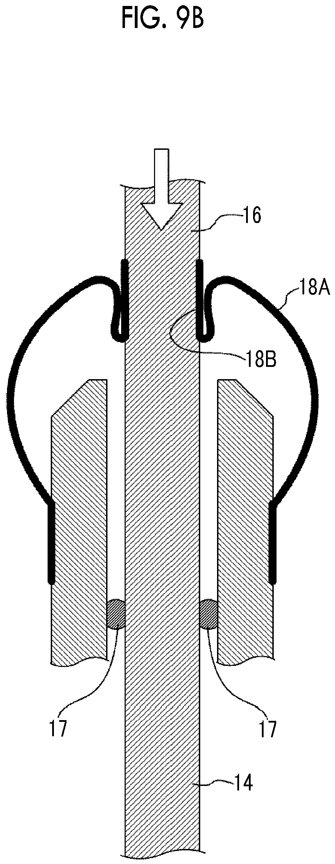

Figs. 9A and 9B are sectional views illustrating the engaging part between the cap 14 and the shaft portion 16 in a state where the sealing member 18 is provided. Fig. 9A illustrates a case where the shaft portion 16 that performs the translational movement is at the ascending position, and Fig. 9B illustrates a case where the shaft portion 16 that performs the translational movement is at the descending position. By sealing the gap formed between the cap 14 and the shaft portion 16 with the sealing member 18, the gap between the cap 14 and the shaft portion 16 can be always in a sealed state, and the risk of contamination of the biological tissue accommodated in the processing container 11 can be suppressed.

It is preferable that the sealing member 18 is composed of a member having elasticity and flexibility. As the material of the sealing member 18, for example, rubber having a rubber hardness of approximately 10 can be suitably used. The sealing member 18 has a small-diameter portion 18B and a large-diameter portion 18C that are arranged along the extension direction of the shaft portion 16. Accordingly, as illustrated in Fig. 9B, the sealing member 18 can be deformed such that the small-diameter portion 18B is buried under the large-diameter portion 18C in accordance with the translational movement of the shaft portion 16. Accordingly, the deformation of the sealing member 18 in accordance with the translational movement of the shaft portion 16 can be minimized, and the stress applied to the sealing member 18 can be suppressed. In addition, the sealing member 18 has a crease 18D (refer to Figs. 7A and 7B) for regulating a folding position in a case where the small-diameter portion 18B is buried under the large-diameter portion 18C. As a result, it is possible to stabilize the deformation of the sealing member 18 in accordance with the translational movement of the shaft portion 16.

Fig. 10 is a diagram illustrating an example of a configuration of the driving unit 20. The driving unit 20 drives the blade 70 via the shaft portion 16. The driving unit 20 includes a translational drive mechanism 21 and a rotational drive mechanism 22. The rotational drive mechanism 22 generates a driving force for rotating the shaft portion 16 with a shaft 23 connected to the shaft portion 16 of the processing module 10 as the rotation axis. The blade 70 attached to the distal end of the shaft portion 16 is rotated in accordance with the rotation of the shaft portion 16. The translational drive mechanism 21 generates a driving force for causing the shaft portion 16 to perform the translational movement along a guide 24 parallel to the extension direction of the shaft portion 16. The blade 70 attached to the distal end of the shaft portion 16 performs the translational movement in the up-down direction with the translational movement of the shaft portion 16. In the first step, the driving unit 20 drives the blade 70 to press the blade 70 against a blade receiver 75. That is, the driving unit 20 drives the blade 70 such that the blade 70 presses and cuts the biological tissue in accordance with the translational movement of the blade 70. The driving unit 20 controls a pressing force in a case of pressing the blade 70 against the blade receiver 75 to a minimum magnitude required for cutting the biological tissue. As a result, it is possible to minimize the wear and deformation of the blade tip, and it is possible to minimize a decrease in sharpness during the division operation.

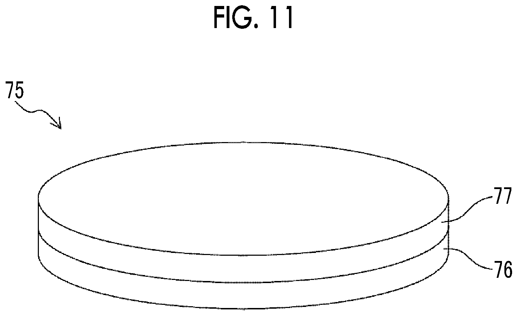

The plate-like blade receiver 75, against which the blade tip of the blade 70 is pressed during the division of the biological tissue, is provided at a bottom portion of the processing container 11 (refer to Fig. 1). Fig. 11 is a perspective view illustrating an example of a configuration of the blade receiver 75. The outer diameter of the blade receiver 75 in the top view is preferably substantially the same as the inner diameter of the bottom portion of the processing container 11. Fig. 11 illustrates the disk-like blade receiver 75. The blade receiver 75 includes a first member 76 having elasticity, and a second member 77 that is stacked on the first member 76 and has higher stiffness than the first member 76. At the time of the division of the biological tissue, the blade 70 is pressed against the second member 77. That is, the blade receiver 75 is installed inside the processing container 11 such that the second member 77 faces upward. As the first member 76, for example, a silicone resin can be used. As the second member 77, glass or metal can be used.

For example, in a case where the blade 70 is attached to the shaft portion 16 in a state of being inclined, in a case where the blade receiver 75 is composed of only the high stiffness member (second member 77), it may be difficult to cut the biological tissue using the entire blade tip of the blade 70. However, since the blade receiver 75 has the first member 76 having elasticity, the blade receiver 75 can be deformed along the inclination of the blade 70. Therefore, it is possible to cut the biological tissue using the entire blade tip of the blade 70, and it is possible to effectively cut the biological tissue. In addition, in a case where fine unevenness is present on the blade tip of the blade 70, in a case where the blade receiver 75 is composed of only the high stiffness member (second member 77), it may be difficult to cut the biological tissue using the entire blade tip of the blade 70. Since the blade receiver 75 has the first member 76 having elasticity and the second member 77 is made into a thin plate thinner than the first member 76, the blade receiver 75 can be deformed along the unevenness of the blade tip. Therefore, it is possible to cut the biological tissue using the entire blade tip of the blade 70, and it is possible to effectively cut the biological tissue. As the second member 77, for example, a thin stainless steel plate (0.1 to 0.3 mm) can be used.

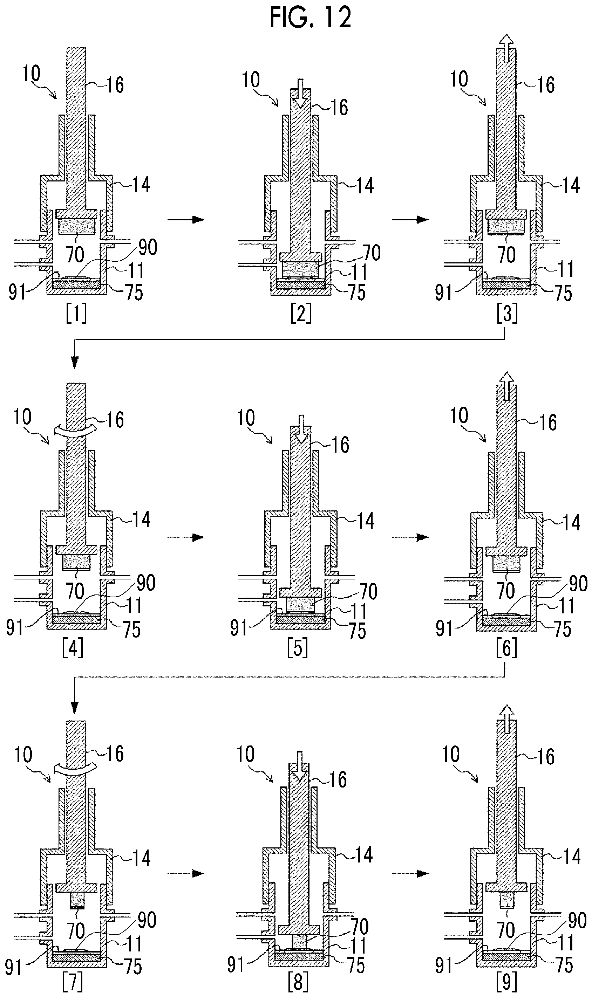

The operation of the processing module 10 in the first step will be described with reference to Fig. 12. Fig. 12 is a diagram illustrating an example of an operation sequence of the processing module 10 in the first step.
[1] The blade receiver 75 is provided at the bottom portion of the processing container 11, and a biological tissue 90 is disposed on the upper surface of the blade receiver 75. A liquid 91 for suppressing the adhesion of the biological tissue 90 to the blade 70 is introduced into the processing container 11 from the first port 13A. The liquid 91 may be, for example, serum or a buffer solution that does not have toxicity to the biological tissue 90.
[2] The driving unit 20 drives the blade 70 such that the blade 70 presses and cuts the biological tissue 90 in accordance with the translational movement of the blade 70. The blade 70 descends to the position where the blade tip hits the blade receiver 75 and presses and cuts the biological tissue 90. In this case, the blade 70 is immersed in the liquid 91. As a result, the adhesion of the biological tissue to the blade 70 is suppressed.
[3] The driving unit 20 raises the blade 70 to separate the blade 70 from the blade receiver 75.
[4] The driving unit 20 rotates the blade 70 about the shaft portion 16 as the rotation axis by a predetermined angle in order to change the cutting position of the biological tissue 90 by the blade 70. That is, the rotation of the blade 70 is performed with a direction of the translational movement of the blade 70 as the rotation axis direction.
[5] The driving unit 20 drives the blade 70 such that the blade 70 presses and cuts the biological tissue 90 in accordance with the translational movement of the blade 70. The blade 70 descends to the position where the blade tip hits the blade receiver 75 and presses and cuts the biological tissue 90. The biological tissue 90 is cut at a position different from the cutting position in [2] by the rotation of the blade 70 in [4].
[6] The driving unit 20 raises the blade 70 to separate the blade 70 from the blade receiver 75.
[7] The driving unit 20 rotates the blade 70 about the shaft portion 16 as the rotation axis by a predetermined angle in order to further change the cutting position of the biological tissue 90 by the blade 70.
[8] The driving unit 20 drives the blade 70 such that the blade 70 presses and cuts the biological tissue 90 in accordance with the translational movement of the blade 70. The blade 70 descends to the position where the blade tip hits the blade receiver 75 and presses and cuts the biological tissue 90. The biological tissue 90 is cut at a position different from the cutting position in [2] and [5] by the rotation of the blade 70 in [7].
[9] The driving unit 20 raises the blade 70 to separate the blade 70 from the blade receiver 75.

As described above, in the first step, the driving unit 20 repeatedly performs the series of operations including an operation of pressing the blade 70 against the blade receiver 75, an operation of separating the blade 70 from the blade receiver 75, and an operation of changing the relative position between the blade 70 and the blade receiver 75. That is, the cell isolation device 1 cuts the biological tissue 90 while changing the position where the blade 70 is pressed against the blade receiver 75. As a result, the biological tissue is divided into small pieces. In a case where the operation of pressing the blade 70 against the blade receiver 75 is performed, the pressing force of the blade 70 is controlled to be a minimum magnitude required for cutting the biological tissue. As a result, it is possible to minimize the wear and deformation of the blade tip, and it is possible to minimize a decrease in sharpness during the division operation.

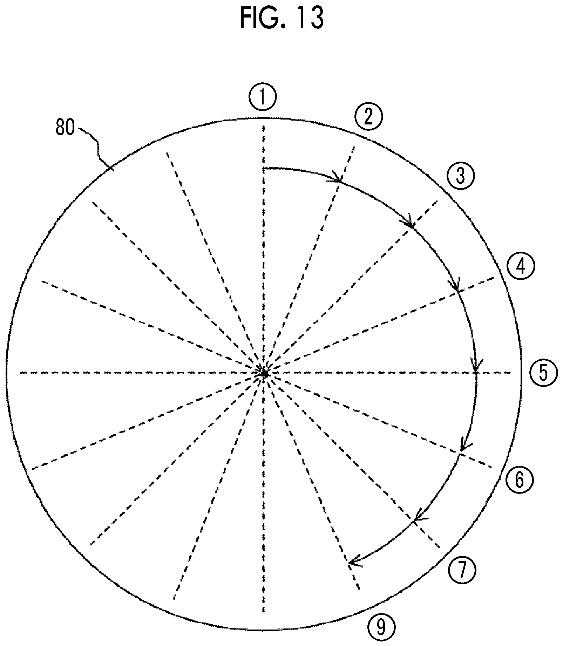

Fig. 13 is a diagram illustrating an example of an aspect of a change in a relative position between the blade 70 and the blade receiver 75. In Fig. 13, broken lines indicate positions where the blade 70 is pressed against the blade receiver 75. In the first step of dividing the biological tissue 90, as illustrated in Fig. 13, the blade 70 is driven such that the pressing position of the blade 70 is changed sequentially by the rotational movement of the blade 70. Accordingly, since the cutting position of the biological tissue 90 can be changed, it is possible to efficiently divide the biological tissue 90. Note that, in the present embodiment, the aspect in which the operation of changing the relative position between the blade 70 and the blade receiver 75 is performed by the rotation of the blade 70 has been exemplified, but the operation of changing the relative position between the blade 70 and the blade receiver 75 may be performed by the rotation of the blade receiver 75 or by the rotation of both the blade 70 and the blade receiver 75.

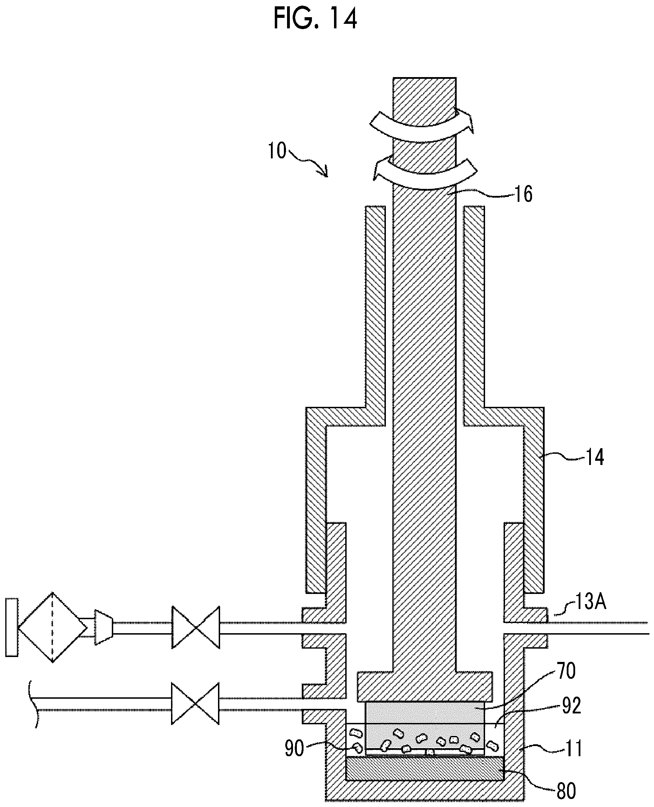

Fig. 14 is a diagram illustrating an example of the operation of the processing module 10 in the second step. In the second step, a first processing liquid 92 for decomposing the biological tissue divided into small pieces is introduced into the processing container 11 from the first port 13A. The biological tissue 90 divided into small pieces is immersed in the first processing liquid 92. The first processing liquid 92 may be, for example, an enzyme solution.

In the second step, the driving unit 20 rotates the blade 70 with the shaft portion 16 as the rotation axis in a state where the blade 70 is immersed in the first processing liquid 92. As a result, the first processing liquid 92 containing the biological tissue 90 divided into small pieces is stirred. That is, the blade 70 functions not only as a cutting blade for dividing the biological tissue 90 but also as a stirring blade for stirring the first processing liquid 92 containing the divided biological tissue 90. By stirring the first processing liquid 92 containing the biological tissue 90, the decomposition of the biological tissue 90 can be promoted. The rotation of the blade 70 may be a twisting operation. The twisting operation is an operation of repeating an operation of rotating the blade 70 clockwise by a predetermined angle (for example, 180°) from an initial position and then rotating the blade 70 counterclockwise to return the blade 70 to the initial position.

As illustrated in Fig. 1, the processing liquid storage portion 30 includes a plurality of storage containers 31A to 31D that store various processing liquids used in the first to third steps. The storage containers 31A to 31D may have a form of a syringe. In the storage container 31A, in the first step, in a case where the biological tissue is divided using the blade 70, a liquid for suppressing the adhesion of the biological tissue to the blade 70 is stored. As the liquid for suppressing the adhesion, serum or a buffer solution can be used. The storage container 31B stores the first processing liquid for decomposing the biological tissue in the second step. As the first processing liquid, for example, an enzyme solution can be used. The storage containers 31C and 31D store the second processing liquid for stopping the decomposition of the biological tissue in the third step. As the second processing liquid, for example, a culture medium can be used.

The switching mechanism 40 switches the liquid or the gas to be introduced into the processing container 11 via the first port 13A. That is, the switching mechanism 40 is a mechanism for selectively introducing various processing liquids or gases (air) stored in the storage containers 31A to 31D, into the processing container 11. The switching mechanism 40 includes a pipe 41 to which each of the storage containers 31A to 31D is connected, switching valves 42A to 42D provided on the pipe 41 to correspond to the storage containers 31A to 31D, and a sterile filter 43 connected to the terminal end of the pipe 41. In a case where the switching valve 42A is in an open state, the processing liquid stored in the storage container 31A flows out to the pipe 41. On the other hand, in a case where the switching valve 42A is in a closed state, the outflow of the processing liquid stored in the storage container 31A to the pipe 41 is stopped. The same applies to the switching valves 42B to 42D. The opening and closing of the switching valves 42A to 42D are controlled by control signals output from the control unit 60. Note that the switching of the switching valves 42A to 42D can also be manually performed.

The liquid feeding portion 50 is provided on a flow channel 51A that connects the processing liquid storage portion 30 and the first port 13A of the processing container 11. The liquid feeding portion 50 transfers various processing liquids stored in the processing liquid storage portion 30 to the processing container 11. The liquid feeding portion 50 may be, for example, a tube pump. For example, by operating the liquid feeding portion 50 with the switching valve 42A in the open state, the processing liquid stored in the first storage container 31A is transferred to the processing container 11. In addition, by operating the liquid feeding portion 50 with all the switching valves 42A to 42D in the closed state, the sterile air introduced from the outside into the flow channel 51A via the sterile filter 43 is transferred to the processing container 11. By transferring the sterile air after transferring the processing liquid, the processing liquid remaining in the tube can be flushed out, and a predetermined amount of the processing liquid can be transferred with high accuracy. The feeding of liquid by the liquid feeding portion 50 is controlled by control signals output from the control unit 60.

A pinch valve 52A is provided on the flow channel 51A connected to the first port 13A, a pinch valve 52B is provided on a flow channel 51B connected to the second port 13B, and a pinch valve 52C is provided on a flow channel 51C connected to the third port 13C. The opening and closing of the pinch valves 52A to 52C are controlled by control signals output from the control unit 60. The flow channel 51B is connected to a processing device (not illustrated) in the next step. The sterile filter 53 is connected to the terminal end of the flow channel 51C.

The control unit 60 controls the driving unit 20, the liquid feeding portion 50, the switching mechanism 40, and the pinch valves 52A to 52C such that the first step of dividing the biological tissue accommodated in the processing container 11 into small pieces by cutting the biological tissue with the blade 70 and the second step of introducing the first processing liquid into the processing container 11 to decompose the divided biological tissue in the processing container 11 are sequentially executed. In addition, in the second step, the control unit controls the driving unit 20 to rotate the blade in a state where the blade 70 is immersed in the first processing liquid. In addition, the control unit 60 controls the liquid feeding portion 50, the switching mechanism 40, and the pinch valves 52A to 52C such that the third step of introducing the second processing liquid into the processing container 11 to stop the decomposition of the biological tissue is executed after the second step. In addition, the control unit 60 controls the liquid feeding portion 50, the switching mechanism 40, and the pinch valves 52A to 52C such that the fourth step of discharging the processing liquid containing the cells isolated from the biological tissue, from the processing container 11 is executed after the third step. In a case where the control unit 60 performs the above-described control, the automation of the treatment required for isolating the cells from the biological tissue is realized.

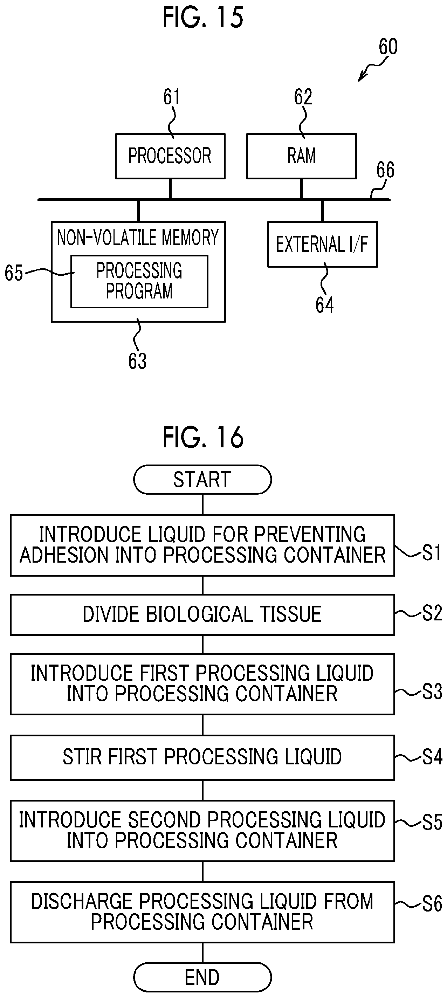

Fig. 15 is a diagram illustrating an example of a hardware configuration of the control unit 60. The control unit 60 includes a processor 61, a random access memory (RAM) 62, a non-volatile memory 63, and an external interface 64. These pieces of hardware are connected to a bus 66. The non-volatile memory 63 is a non-volatile storage medium, such as a flash memory. A processing program 65 is stored in the non-volatile memory 63. The RAM 62 is a work memory for the processor 61 to execute processing. The processor 61 reads out the processing program 65 from the non-volatile memory 63, and then expands the processing program 65 in the RAM 62 to execute the processing program 65. The external interface 64 is an interface to which the driving unit 20, the liquid feeding portion 50, the switching mechanism 40, and the pinch valves 52A to 52C, which are control targets by the control unit 60, are connected.

The above-described control by the control unit 60 is realized by the processor 61 executing the processing program 65. Fig. 16 is a flowchart illustrating an example of a flow of processing that is executed by the processor 61 executing the processing program 65. The processing program 65 is executed, for example, in a case where a predetermined operation for instructing the start of the processing is performed with respect to the cell isolation device 1. Note that the biological tissues as the processing target are accommodated in the processing container 11, and various processing liquids are stored in the storage containers 31A to 31D.

In step S1, the control unit 60 controls the pinch valves 52A and 52C to be in the open state, and controls the pinch valve 52B to be in the closed state. In addition, the control unit 60 controls the switching valve 42A to be in the open state, and operates the liquid feeding portion 50. As a result, the liquid (for example, serum) stored in the storage container 31A is introduced from the first port 13A into the processing container 11. This liquid is a liquid for suppressing the adhesion of the biological tissue to the blade 70. In a case where a predetermined amount of the liquid is introduced into the processing container 11, the control unit 60 controls each of the pinch valve 52A and the switching valve 42A to be in the closed state, and stops the operation of the liquid feeding portion 50.

In step S2, the control unit 60 controls the driving unit 20 to perform the division operation of the biological tissue. Accordingly, as illustrated in Fig. 12, the driving unit 20 repeatedly performs the series of operations including an operation of pressing the blade 70 against the blade receiver 75, an operation of separating the blade 70 from the blade receiver 75, and an operation of changing the relative position between the blade 70 and the blade receiver 75. The biological tissue is divided into small pieces by the excision by the translational movement of the blade 70. In a case where a predetermined time has elapsed after the start of the division operation, the control unit 60 stops the operation of the driving unit 20. The processing of steps S1 and S2 corresponds to the first step.

In step S3, the control unit 60 controls the pinch valves 52A and 52C to be in the open state, and controls the pinch valve 52B to be in the closed state. In addition, the control unit 60 controls the switching valve 42B to be in the open state, and operates the liquid feeding portion 50. As a result, the first processing liquid (for example, the enzyme solution) stored in the storage container 31B is introduced from the first port 13A into the processing container 11. In a case where a predetermined amount of the first processing liquid is introduced into the processing container 11, the control unit 60 controls each of the pinch valve 52A and the switching valve 42B to be in the closed state, and stops the operation of the liquid feeding portion 50.

In step S4, the control unit 60 controls the driving unit 20 to perform a stirring operation of the first processing liquid containing the divided biological tissue. The driving unit 20 stirs the first processing liquid containing the biological tissue divided into small pieces by rotating the blade 70 with the shaft portion 16 as the rotation axis in a state where the blade 70 is immersed in the first processing liquid. The rotation of the blade 70 may be a twisting operation. As a result, the biological tissue divided into small pieces is decomposed, and the cells contained in the biological tissue are isolated. In a case where a predetermined time has elapsed after the start of the stirring operation, the control unit 60 stops the operation of the driving unit 20. The processing of steps S3 and S4 corresponds to the second step.

In step S5, the control unit 60 controls the pinch valves 52A and 52C to be in the open state, and controls the pinch valve 52B to be in the closed state. In addition, the control unit 60 controls the switching valve 42C to be in the open state, and operates the liquid feeding portion 50. As a result, the second processing liquid (for example, the culture medium) stored in the storage container 31C is introduced from the first port 13A into the processing container 11. As a result, the first processing liquid introduced into the processing container 11 is diluted, and the decomposition of the biological tissue is stopped. As a result, the damage to the cells due to excessive decomposition can be suppressed. In a case where a predetermined amount of the second processing liquid is introduced into the processing container 11, the control unit 60 controls each of the pinch valve 52A and the switching valve 42C to be in the closed state, and stops the operation of the liquid feeding portion 50. The processing of step S5 corresponds to the third step.

In step S6, the control unit 60 controls the pinch valves 52A and 52B to be in the open state, and controls the pinch valve 52C to be in the closed state. In addition, the control unit 60 controls all the switching valves 42A to 42D to be in the closed state, and operates the liquid feeding portion 50. Accordingly, the sterile air introduced into the flow channel 51A from the outside via the sterile filter 43 is introduced from the first port 13A into the processing container 11, and the pressure in the processing container 11 is increased. As a result, the processing liquid containing the cells isolated from the biological tissue is discharged from the second port 13B, and is sent to the next step through the flow channel 51B. The processing of step S6 corresponds to the fourth step.

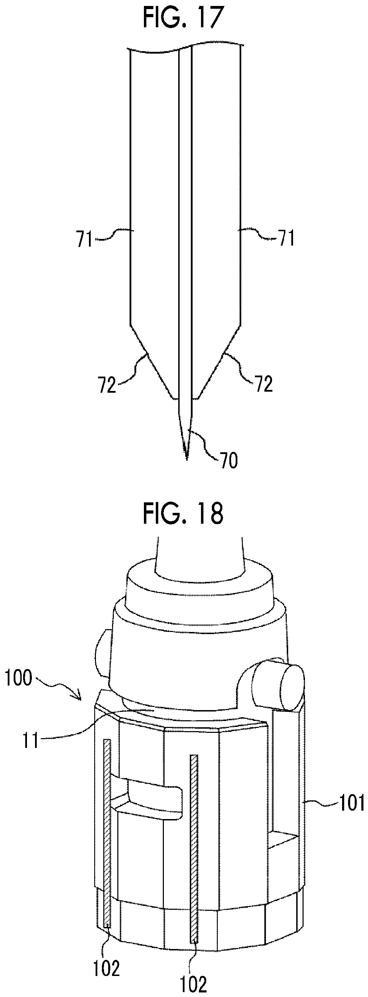

The cell isolation device 1 may have the following configuration. As illustrated in Fig. 17, the cell isolation device 1 may include an adhesion suppressing member 71 for suppressing the adhesion of the biological tissue to the blade 70 in a case of dividing the biological tissue. The adhesion suppressing member 71 is provided on the side surface of the blade 70, and has an inclined part 72 that is inclined toward the blade tip of the blade 70. The inclined part 72 acts to suppress the crawling of the biological tissue cut by the blade 70, to the side surface of the blade 70. As a result, the adhesion of the biological tissue to the blade 70 is suppressed, and the biological tissue can be appropriately cut. Note that, instead of the adhesion suppressing member 71, a sheet having water repellency may be provided on the side surface of the blade 70 or a coating agent having water repellency may be coated on the side surface of the blade 70.

The cell isolation device 1 may include a temperature control mechanism that controls the first processing liquid accommodated in the processing container 11 to an optimum temperature in the second step. Fig. 18 is a perspective view illustrating an example of a configuration of a temperature control mechanism 100. The temperature control mechanism 100 includes a cup-shaped holder 101 that surrounds an outer periphery of the processing container 11, and a heater 102 that is built in the holder 101. The holder 101 is composed of a metal such as aluminum having a relatively high thermal conductivity. Heat emitted from the heater 102 is substantially uniformly diffused in the entire holder 101. The surface of the processing container 11 is in contact with the inner wall of the holder 101, and the first processing liquid introduced into the processing container 11 is heated via the holder 101. The surface temperature of the holder 101 is monitored using a thermocouple (not illustrated) screwed to the surface of the holder 101. As the thermocouple, for example, a terminal type thermocouple may be used. The heater 102 is, for example, a cartridge heater, and is controlled such that the monitored surface temperature of the holder 101 is a predetermined temperature. As a result, the first processing liquid accommodated in the processing container 11 is controlled to the optimum temperature. By controlling the first processing liquid to the optimum temperature, the decomposition of the biological tissue can be promoted, and a processing time in the second step can be shortened as compared with a case where the temperature control is not performed. The optimum temperature is appropriately set according to the type of the biological tissue as the processing target, the type of the first processing liquid to be used, and the like. For example, in a case of decomposing the synovial membrane with the enzyme solution, it is preferable to perform the temperature control such that the enzyme solution is maintained at a temperature of about 37°C. In addition, the cell isolation device 1 may have a mechanism that shakes the processing container 11 in the second step. In the second step, by shaking the processing container 11, the decomposition of the biological tissue can be promoted, and the processing time in the second step can be further shortened.

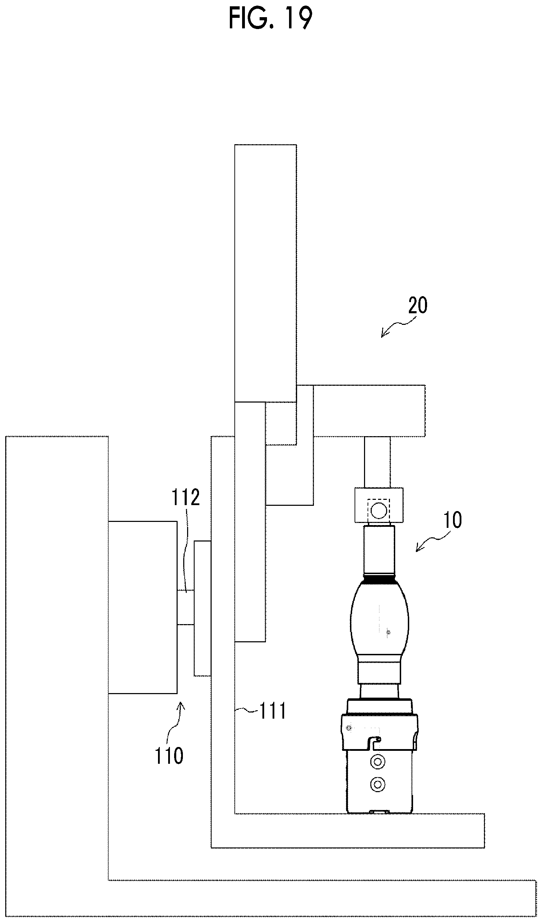

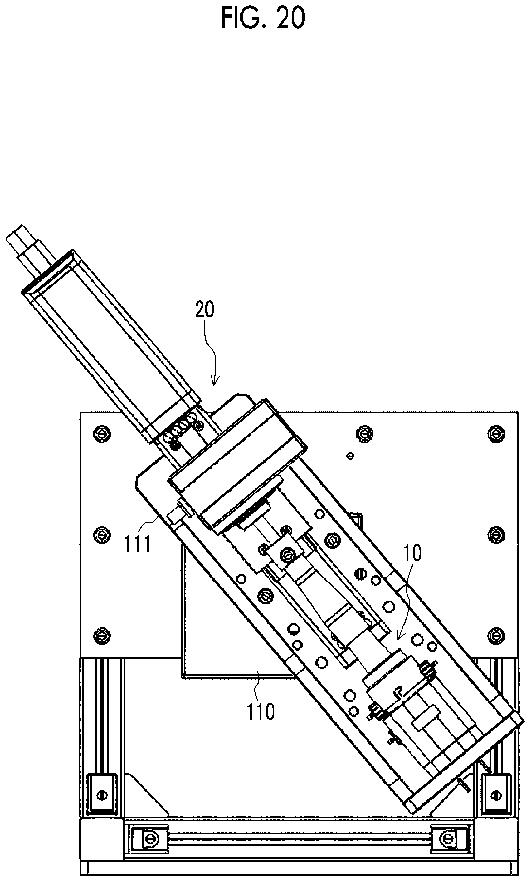

The cell isolation device 1 may include an inclination mechanism that inclines the processing container 11 such that the processing liquid is discharged from the second port 13B in the fourth step. Fig. 19 is a side view and a front view illustrating an example of the configuration of an inclination mechanism 110. The cell isolation device 1 includes a frame 111 that fixes a relative position between the driving unit 20 and the processing module 10. The inclination mechanism 110 generates a driving force for rotating the frame 111 by using the shaft 112 connected to the rear surface of the frame 111 as the rotation axis. As illustrated in Fig. 20, the driving unit 20 and the processing module 10 are integrally inclined with the rotation of the frame 111. The processing container 11 is inclined such that the second port 13B is positioned at the lowermost portion of the processing container 11. As a result, since the discharge of the processing liquid from the second port 13B is promoted, the remaining of the cells isolated from the biological tissue, in the processing container 11 can be suppressed. That is, the processing liquid containing the cells obtained by the cell isolation processing can be sent to the next step with a minimum loss.

As described above, the cell isolation device 1 according to the present embodiment includes the blade 70 for cutting the biological tissue, the blade receiver 75 against which the blade 70 is pressed, and the driving unit 20 that drives the blade 70 such that the blade 70 is pressed against the blade receiver 75. That is, the driving unit 20 drives the blade 70 such that the blade 70 presses and cuts the biological tissue in accordance with the translational movement of the blade 70. As a result, for example, the damage to the cells can be reduced as compared with a case of driving the blade to crush the biological tissue. That is, with the cell isolation device 1 according to the present embodiment, it is possible to ensure a high survival rate in the cells obtained by executing the cell isolation processing. **In** addition, even in a fibrous tissue such as a synovial membrane that is difficult to cut with the rotary blade, it is possible to reliably cut the fibrous tissue. Note that, in the present embodiment, although a case where the biological tissue is pressed and cut by the reciprocating motion of the blade 70 in a straight line has been exemplified, the blade 70 may press and cut the biological tissue by performing the reciprocating motion on an arc. For example, the biological tissue may be pressed and cut by attaching the blade 70 to the other end of an arm of which one end is fixed to the rotation axis and by rotating the arm around the rotation axis to bring the blade 70 downward.

The driving unit 20 drives the blade 70 to change the cutting position of the biological tissue by the blade 70. Accordingly, it is possible to efficiently divide the biological tissue. The driving unit 20 changes the cutting position by the rotation operation in which the direction of the translational movement of the blade 70 is the rotation axis direction. Accordingly, as compared with a case where the cutting position is changed by the parallel movement of the blade 70, the size of the movement space of the blade 70 can be reduced, and the device can be made compact.

The driving unit 20 repeatedly performs the series of operations including an operation of pressing the blade 70 against the blade receiver 75, an operation of separating the blade 70 from the blade receiver 75, and an operation of changing the relative position between the blade 70 and the blade receiver 75. As a result, for example, the damage to the cells can be reduced as compared with a case of driving the blade to crush the biological tissue. **In** addition, even in a fibrous tissue that is difficult to cut with the rotary blade, it is possible to reliably cut the fibrous tissue. The driving unit 20 controls a pressing force in a case of pressing the blade 70 against the blade receiver 75 to a minimum magnitude required for cutting the biological tissue. As a result, it is possible to minimize the wear and deformation of the blade tip, and it is possible to minimize a decrease in sharpness during the division operation.

The blade receiver 75 includes the first member 76 having elasticity, and the second member 77 that is stacked on the first member 76 and has higher stiffness than the first member 76, and the blade 70 is pressed against the second member 77. Since the blade receiver 75 has the first member 76 having elasticity, the blade receiver 75 can be deformed along the inclination of the blade 70. Therefore, it is possible to cut the biological tissue using the entire blade tip of the blade 70, and it is possible to effectively and finely cut the biological tissue. The second member 77 may be thinner than the first member 76. Accordingly, the blade receiver 75 can be deformed along the unevenness of the blade tip, and the biological tissue can be effectively and finely cut.

The cell isolation device 1 may include the adhesion suppressing member 71 for suppressing the adhesion of the biological tissue to the blade 70. The adhesion suppressing member 71 is provided on the side surface of the blade 70, and has an inclined part 72 that is inclined toward the blade tip of the blade 70. As a result, the adhesion of the biological tissue to the blade 70 is suppressed, and the biological tissue 90 can be appropriately cut.

The cell isolation device includes the processing container 11 that can accommodate the biological tissue, and the liquid feeding portion 50 that transfers the first processing liquid for decomposing the biological tissue cut by the blade 70 in the processing container 11, into the processing container 11. Since the division and decomposition of the biological tissue are performed using the same processing container 11, it is possible to reduce the loss of cells as compared with a case where the division and decomposition are performed using separate containers.

The processing container 11 includes the first port 13A for introducing the liquid or the gas containing the first processing liquid into the processing container 11, the second port 13B for discharging the processed liquid from the processing container 11, and the third port 13C for an air vent. As a result, various processing liquids or gases (air) can be put into and taken out of the processing container 11. The cell isolation device 1 includes the switching mechanism 40 that switches the liquid or the gas to be introduced into the processing container 11 via the first port 13A. Accordingly, various processing liquids and gases can be selectively introduced into the processing container 11, and it is possible to cope with the automation of the processing.

The cell isolation device 1 may include the inclination mechanism 110 that inclines the processing container 11 such that the discharge of the processing liquid from the second port 13B is promoted. As a result, it is possible to suppress the remaining of the cells isolated from the biological tissue in the inside of the processing container 11. That is, it is possible to minimize the loss of cells and to send the processing liquid containing the isolated cells to the next step.

The cell isolation device 1 includes the tubular cap 14 that covers the processing container 11, and the shaft portion 16 which is inserted into the cap 14, of which one end is attached to the blade 70 and the other end is attached to the driving unit. Accordingly, the processing container 11 can be brought into a sealed state, and it is possible to suppress the risk of contamination of the biological tissue accommodated in the processing container 11.

The blade 70 is attached to the shaft portion 16 via the grip portion 80 that grips the blade 70 by point contact or line contact with respect to the blade 70. Accordingly, it is possible to suppress the retention of the processing liquid in the contact portion between the grip portion 80 and the blade and to prevent the corrosion of the blade 70.

The cell isolation device 1 has the sealing member 18 that seals a gap formed between the cap 14 and the shaft portion 16. Accordingly, the gap between the cap 14 and the shaft portion 16 can be always brought into a sealed state, and it is possible to suppress the risk of contamination of the biological tissue accommodated in the processing container 11. The sealing member 18 is composed of a member having flexibility. Accordingly, the sealing member 18 can be deformed in accordance with the translational movement of the shaft portion 16. The sealing member 18 has the small-diameter portion 18B and the large-diameter portion 18C that are arranged along the extension direction of the shaft portion 16, and the small-diameter portion 18B is buried under the large-diameter portion 18C in accordance with the translational movement along the extension direction of the shaft portion 16. Accordingly, the deformation of the sealing member 18 in accordance with the translational movement of the shaft portion 16 can be minimized, and the stress applied to the sealing member 18 can be suppressed. The sealing member 18 has a crease for regulating a folding position in a case where the small-diameter portion 18B is buried under the large-diameter portion 18C. As a result, it is possible to stabilize the deformation of the sealing member 18 in accordance with the translational movement of the shaft portion 16.

The cell isolation device 1 may include the temperature control mechanism 100 that controls the first processing liquid accommodated in the processing container 11 to an optimum temperature. As a result, the decomposition of the biological tissue can be promoted, and a processing time in the second step can be shortened as compared with a case where the temperature control is not performed.

The cell isolation device 1 includes the control unit 60 that controls the driving unit 20 and the liquid feeding portion 50 such that the first step of dividing the biological tissue accommodated in the processing container 11 into small pieces by cutting the biological tissue with the blade 70 and the second step of introducing the first processing liquid into the processing container 11 to decompose the divided biological tissue in the processing container are sequentially executed. In a case where the control unit 60 performs the above-described control, the automation of the treatment required for isolating the cells from the biological tissue is realized.

In the second step, the control unit 60 controls the driving unit 20 to rotate the blade in a state where the blade 70 is immersed in the first processing liquid. As a result, the first processing liquid containing the biological tissue divided into small pieces is stirred. That is, the blade 70 can function not only as the cutting blade for dividing the biological tissue but also as the stirring blade for stirring the first processing liquid containing the divided biological tissue. By stirring the first processing liquid containing the biological tissue, the decomposition of the biological tissue can be promoted.

The control unit 60 controls the liquid feeding portion 50 such that the third step of introducing the second processing liquid into the processing container 11 to stop the decomposition of the biological tissue is executed after the second step. As a result, the damage to the cells due to excessive decomposition can be suppressed.

The control unit 60 controls the liquid feeding portion 50 such that the fourth step of discharging the processing liquid containing the cells isolated from the biological tissue, from the processing container 11 is executed after the third step. As a result, the processing of sending the cells obtained by the cell isolation processing to the next step is automated.

A cell isolation method according to the embodiment of the disclosed technology is performed by the cell isolation device 1. That is, the cell isolation method according to the embodiment of the disclosed technology is a cell isolation method of isolating cells from the biological tissue, and includes performing cutting processing of pressing and cutting the biological tissue disposed on the blade receiver 75 by pressing the blade 70 against the blade receiver 75 by a mechanical operation. As a result, for example, the damage to the cells can be reduced as compared with a case of driving the blade to crush the biological tissue. That is, with the cell isolation method according to the present embodiment, it is possible to ensure a high survival rate in the cells obtained by executing the cell isolation processing. In addition, even in a fibrous tissue such as a synovial membrane that is difficult to cut with the rotary blade, it is possible to reliably cut the fibrous tissue.

The cutting processing is performed inside the sealed processing container 11. As a result, it is possible to suppress the risk of contamination of the biological tissue by the contamination source from the outside. The cell isolation method according to the embodiment of the disclosed technology includes cutting the biological tissue while changing the cutting position of the biological tissue by the blade 70 in the cutting processing. Accordingly, it is possible to efficiently cut the biological tissue. The cell isolation method according to the embodiment of the disclosed technology includes pressing and cutting the biological tissue by the translational movement of the blade 70. Accordingly, in a case where the cutting processing of the biological tissue is performed by the mechanical operation, the device can be configured to be compact. The pressing force in a case of pressing the blade against the blade receiver is controlled to a minimum magnitude required for cutting the biological tissue. As a result, it is possible to minimize the wear and deformation of the blade tip, and it is possible to minimize a decrease in sharpness during the division operation.

The cell isolation method according to the embodiment of the disclosed technology includes the first step of dividing the biological tissue accommodated in the processing container 11 into small pieces by the cutting processing and the second step of introducing the first processing liquid into the processing container 11 to decompose the small pieces of the biological tissue in the processing container 11. Since the division and decomposition of the biological tissue are performed using the same processing container 11, it is possible to reduce the loss of cells as compared with a case where the division and decomposition are performed using separate containers.

The cell isolation method according to the embodiment of the disclosed technology includes, in the first step, dividing the biological tissue while changing the cutting position of the biological tissue by the blade 70. Accordingly, it is possible to efficiently divide the biological tissue.

The cell isolation method according to the embodiment of the disclosed technology includes, in the first step, dividing the biological tissue in a state where the blade 70 is immersed in the liquid introduced to the processing container 11. As a result, it is possible to suppress the adhesion of the biological tissue to the blade 70.

The cell isolation method according to the embodiment of the disclosed technology includes, in the second step, rotating the blade 70 in a state where the blade 70 is immersed in the first processing liquid. As a result, the first processing liquid containing the biological tissue divided into small pieces is stirred. That is, the blade 70 can function not only as the cutting blade for dividing the biological tissue but also as the stirring blade for stirring the first processing liquid containing the divided biological tissue. By stirring the first processing liquid containing the biological tissue, the decomposition of the biological tissue can be promoted.

The cell isolation method according to the embodiment of the disclosed technology may include controlling the first processing liquid to an optimum temperature in the second step. As a result, the decomposition of the biological tissue can be promoted, and a processing time in the second step can be shortened as compared with a case where the temperature control is not performed.

In the cell isolation method according to the embodiment of the disclosed technology, in the first step and the second step, the inside of the processing container 11 is maintained in a sealed state. As a result, it is possible to suppress the risk of contamination of the biological tissue accommodated in the processing container 11, by the contamination source from the outside.

The cell isolation method according to the embodiment of the disclosed technology includes the third step of introducing the second processing liquid into the processing container 11 to stop the decomposition of the biological tissue after the second step. As a result, the damage to the cells due to excessive decomposition can be suppressed.

The cell isolation method according to the embodiment of the disclosed technology includes repeatedly performing, in a state where the biological tissue is disposed on the blade receiver 75, the series of operations including an operation of pressing the blade 70 against the blade receiver 75, an operation of separating the blade 70 from the blade receiver 75, and an operation of changing the relative position between the blade 75 and the blade receiver 75. As a result, for example, the damage to the cells can be reduced as compared with a case of driving the blade to crush the biological tissue. In addition, even in a fibrous tissue that is difficult to cut with the rotary blade, it is possible to reliably cut the fibrous tissue.

### [Examples]

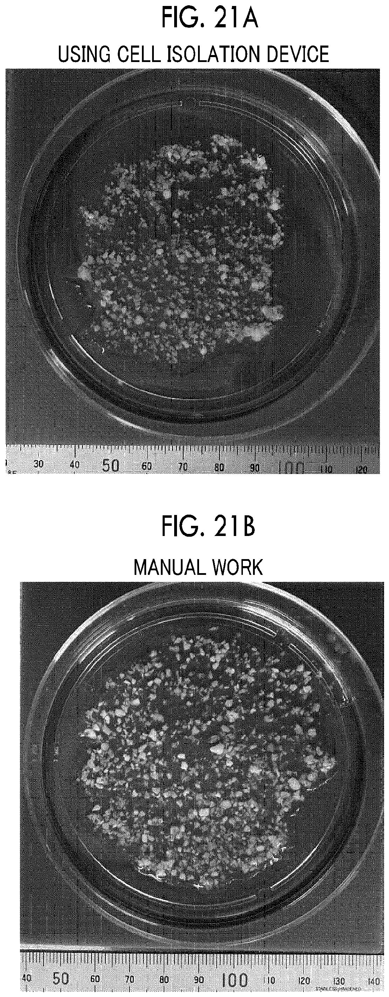

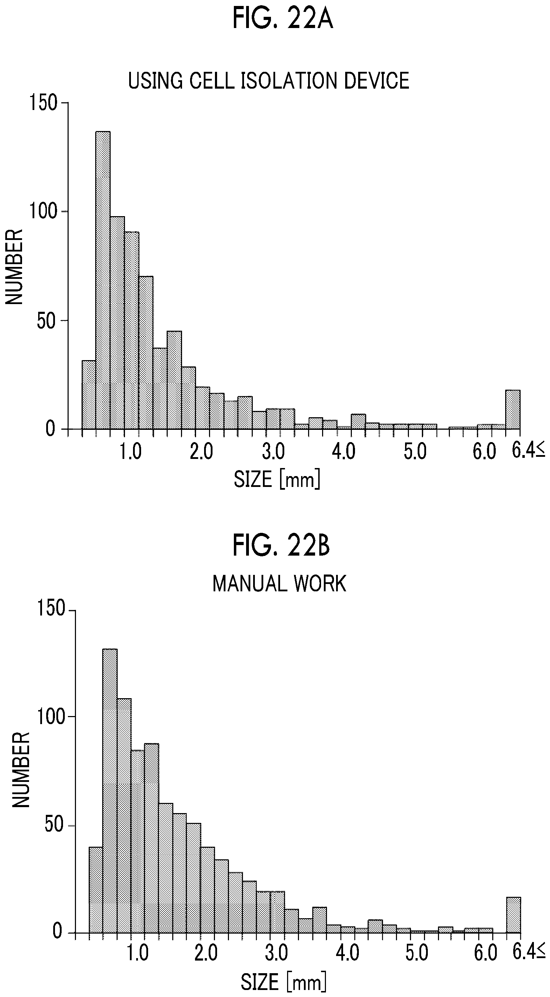

Fig. 21A is an image of a synovial membrane divided into small pieces by using the cell isolation device 1 according to the embodiment of the disclosed technology. Fig. 21B is an image of the synovial membrane divided into small pieces by a manual work. Fig. 22A is a histogram illustrating a size distribution of the synovial membrane divided into small pieces by using the cell isolation device 1 according to the embodiment of the disclosed technology.

Fig. 22B is a histogram illustrating a size distribution of the synovial membrane divided into small pieces by a manual work in the related art. In a case of the manual work in the related art, the synovial membrane having a size of 2 mm square or less was 87.4%. On the other hand, in a case of using the cell isolation device 1 according to the embodiment of the disclosed technology, the synovial membrane having a size of 2 mm square or less was 89.5%. With the cell isolation device 1 according to the embodiment of the disclosed technology, it is possible to divide the synovial membrane in the same manner as in a case of the manual work in the related art.

The upper row of Fig. 23 is an image illustrating a state of decomposition of the synovial membrane by the manual work in the related art, and the lower row of Fig. 23 is an image illustrating a state of decomposition of the synovial membrane by the cell isolation device 1 according to the embodiment of the disclosed technology. With the cell isolation device 1 according to the embodiment of the disclosed technology, it is possible to perform the decomposition processing in the same manner as in a case of the manual work in the related art.

Fig. 24 is a graph illustrating a yield and a viable cell percentage of cells in a case of the manual work in the related art and in a case of using the cell isolation device 1 according to the embodiment of the disclosed technology. With the cell isolation device 1 according to the embodiment of the disclosed technology, it is possible to obtain the same viable cell yield and viable cell percentage as those in a case of the manual work in the related art.

Fig. 25 is an image illustrating a state of the synovial membrane in a case where the division processing of the synovial membrane is performed using the rotary blade, as disclosed in JP2013-011603A. In a case where the synovial membrane is divided using the rotary blade, fibers of the synovial membrane are entangled with the rotary blade to be stretched. In this way, in a case where the rotary blade is used, since the tissues are stretched or crushed, the cells are damaged, and the survival rate of cells is expected to be low.

The disclosure of Japanese Patent Application No. 2022-047028 is incorporated herein by reference in its entirety.

All literatures, patent applications, and technical standards described in the present specification are incorporated in the present specification by reference to the same extent as in a case where the individual literatures, patent applications, and technical standards are specifically and individually stated to be incorporated by reference.

## Claims

1. A cell isolation method of isolating a cell from a biological tissue, the cell isolation method comprising:
performing cutting processing of pressing a blade against a blade receiver and pressing and cutting the biological tissue disposed on the blade receiver, by a mechanical operation.

2. The cell isolation method according to claim 1,
wherein the cutting processing is performed inside a sealed container.

3. The cell isolation method according to claim 1 or 2,
wherein in the cutting processing, the biological tissue is cut while a cutting position of the biological tissue by the blade is changed.

4. The cell isolation method according to any one of claims 1 to 3,
wherein the biological tissue is pressed and cut by a translational movement of the blade.

5. The cell isolation method according to any one of claims 1 to 4, further comprising:
a first step of dividing the biological tissue accommodated in a container into small pieces by the cutting processing; and
a second step of introducing a first processing liquid into the container to decompose the small pieces of the biological tissue in the container.

6. The cell isolation method according to claim 5,
wherein in the first step, the biological tissue is divided while a cutting position of the biological tissue by the blade is changed.

7. The cell isolation method according to claim 5 or 6,
wherein in the first step, the biological tissue is divided in a state where the blade is immersed in a liquid introduced into the container.

8. The cell isolation method according to any one of claims 5 to 7,
wherein in the second step, the blade is rotated in a state where the blade is immersed in the first processing liquid.

9. The cell isolation method according to any one of claims 5 to 8,
wherein in the second step, the first processing liquid is controlled to an optimum temperature.

10. The cell isolation method according to any one of claims 5 to 9,
wherein in the first step and the second step, an inside of the container is maintained in a sealed state.

11. The cell isolation method according to any one of claims 5 to 10, further comprising:
a third step of introducing a second processing liquid into the container to stop decomposition of the biological tissue after the second step.

12. The cell isolation method according to any one of claims 1 to 11,
wherein in a state where the biological tissue is disposed on the blade receiver, a series of operations including an operation of pressing the blade against the blade receiver, an operation of separating the blade from the blade receiver, and an operation of changing a relative position between the blade and the blade receiver is repeatedly performed.

13. The cell isolation method according to any one of claims 1 to 12,
wherein a pressing force in a case of pressing the blade against the blade receiver is controlled to a minimum magnitude required for cutting the biological tissue.

14. A cell isolation device for isolating a cell from a biological tissue, the cell isolation device comprising:
a blade for cutting the biological tissue;
a blade receiver against which the blade is pressed; and
a driving unit that drives the blade such that the blade is pressed against the blade receiver.

15. The cell isolation device according to claim 14,
wherein the driving unit drives the blade such that the biological tissue is pressed and cut by a translational movement of the blade.

16. The cell isolation device according to claim 15,
wherein the driving unit drives the blade such that a cutting position of the biological tissue by the blade is changed.

17. The cell isolation device according to claim 16,
wherein the driving unit changes the cutting position by a rotation operation in which a direction of the translational movement of the blade is a rotation axis direction.

18. The cell isolation device according to claim 16 or 17,
wherein the driving unit repeatedly performs a series of operations including an operation of pressing the blade against the blade receiver, an operation of separating the blade from the blade receiver, and an operation of changing a relative position between the blade and the blade receiver.

19. The cell isolation device according to any one of claims 14 to 18,
wherein the blade receiver includes a first member having elasticity, and a second member that is stacked on the first member and has stiffness higher than the first member, and
the blade is pressed against the second member.

20. The cell isolation device according to claim 19,
wherein the second member is thinner than the first member.

21. The cell isolation device according to any one of claims 14 to 20, further comprising:
an adhesion suppressing member for suppressing adhesion of the biological tissue to the blade.

22. The cell isolation device according to claim 21,
wherein the adhesion suppressing member has an inclined part that is provided on a side surface of the blade and is inclined toward a blade tip of the blade.

23. The cell isolation device according to any one of claims 14 to 22, further comprising:
a container in which the biological tissue is accommodated; and
a liquid feeding portion that transfers a first processing liquid for decomposing the biological tissue cut by the blade in the container, to the container.

24. The cell isolation device according to claim 23,
wherein the container includes
a first port for introducing a liquid or a gas containing the first processing liquid into the container,
a second port for discharging a processed liquid from the container, and
a third port for an air vent.

25. The cell isolation device according to claim 24, further comprising:
a switching mechanism that switches the liquid or the gas to be introduced into the container via the first port.

26. The cell isolation device according to claim 24 or 25, further comprising:
a mechanism that inclines the container such that discharge of a processing liquid from the second port is promoted.

27. The cell isolation device according to any one of claims 23 to 26, further comprising:
a tubular cap that covers the container; and
a shaft portion that is inserted into the cap, and of which one end is attached to the blade and the other end is attached to the driving unit.

28. The cell isolation device according to claim 27,
wherein the blade is attached to the shaft portion via a grip portion that grips the blade by point contact or line contact with respect to the blade.

29. The cell isolation device according to claim 27 or 28, further comprising:
a sealing member that seals a gap formed between the cap and the shaft portion.

30. The cell isolation device according to claim 29,
wherein the sealing member is composed of a member having flexibility.

31. The cell isolation device according to claim 29 or 30,
wherein the sealing member has a large-diameter portion and a small-diameter portion that are arranged along an extension direction of the shaft portion, and
the small-diameter portion is buried under the large-diameter portion in accordance with a translational movement along the extension direction of the shaft portion.

32. The cell isolation device according to claim 31,
wherein the sealing member has a crease for regulating a folding position in a case where the small-diameter portion is buried under the large-diameter portion.

33. The cell isolation device according to any one of claims 23 to 32, further comprising:
a temperature control mechanism that controls the first processing liquid accommodated in the container to an optimum temperature.

34. The cell isolation device according to any one of claims 23 to 33, further comprising:
a control unit that controls the driving unit and the liquid feeding portion such that a first step of dividing the biological tissue accommodated in the container into small pieces by cutting the biological tissue using the blade, and a second step of introducing the first processing liquid into the container to decompose the divided biological tissue in the container are sequentially executed.

35. The cell isolation device according to claim 34,
wherein the control unit controls the driving unit such that the blade is rotated in a state where the blade is immersed in the first processing liquid, in the second step.

36. The cell isolation device according to claim 34 or 35,
wherein the control unit controls the liquid feeding portion such that a third step of introducing a second processing liquid into the container to stop decomposition of the biological tissue is executed after the second step.

37. The cell isolation device according to claim 36,
wherein the control unit controls the liquid feeding portion such that a fourth step of discharging a processing liquid containing the cell isolated from the biological tissue, from the container is executed after the third step.

38. The cell isolation device according to any one of claims 14 to 37,
wherein the driving unit controls a pressing force in a case of pressing the blade against the blade receiver to a minimum magnitude required for cutting the biological tissue.
